# EUROPÄISCHE PATENTSCHRIFT

(11) **EP 2 392 608 B1**
(45) Veröffentlichungstag und Bekanntmachung des Hinweises auf die Patenterteilung: **18.11.2020**
(21) Anmeldenummer: 11164513.1
(22) Anmeldetag: 03.05.2011
(51) Int. Cl.: C08G 77/38, C08G 77/388, A61K 8/89, B01F 17/00, C11D 3/37, D06M 15/643

(54) **Verfahren zur Herstellung von organischen Siliciumverbindungen**
Method for producing organic silicon compounds
Procédé destiné à la modification de polysiloxanes organiques

(30) Priorität: 07.06.2010 DE 102010029723
(43) Veröffentlichungstag der Anmeldung: 07.12.2011
(73) Patentinhaber: Evonik Operations GmbH, 45128 Essen (DE)
(72) Erfinder: Knott, Wilfried, 45355 Essen (DE); Klein, Klaus-Dieter, Dr., 45481 Mülheim an der Ruhr (DE)
(74) Vertreter: Evonik Patent Association

(56) Entgegenhaltungen:
- EP-A1- 0 075 703
- EP-A1- 0 940 422
- EP-A1- 1 380 588
- EP-A1- 1 382 630
- US-A1- 2010 022 435
- YILGOR I ET AL: "POLYSILOXANE CONTAINING COPOLYMERS: A SURVEY OF RECENT DEVELOPMENTS", ADVANCED IN POLYMER SCIENCE, BERLIN, DE, 1. Januar 1988 (1988-01-01), Seiten 1-86, XP000571005,
- Cas: "Platinum, di-.mu.-chlorodichlorobis[(1,2-.eta.)-cycl ohexene]di-" In: "Platinum, di-.mu.-chlorodichlorobis[(1,2-.eta.)-cycl ohexene]di-", 15 May 2018 (2018-05-15), XP055475488, * the whole document *

## Beschreibung

Die Erfindung betrifft ein Verfahren zur Addition von Siloxanen, welche SiH-Gruppen aufweisen an organischen Verbindungen mit olefinischen Doppelbindungen in Gegenwart von Di-µ-chloro-bis(1,2-η)-cyclohexen platin(II)chlorid als Katalysator.
Die Erfindung betrifft insbesondere ein Verfahren zur Addition SiH-Gruppen aufweisender Siloxane an Verbindungen die olefinische Doppelbindungen aufweisen, wie beispielsweise olefinisch ungesättigte Verbindungen ausgewählt aus der Gruppe von Estern, Aminen, Amiden, Alkoholen, Ethern oder Kohlenwasserstoffen.

SiC-verknüpfte, organomodifizierte Siloxane, speziell Polyethersiloxane, stellen mit ihrem weit einstellbaren Tensidverhalten eine industriell sehr wichtige Stoffklasse dar. Der etablierte Weg zur Herstellung dieser Substanzen liegt in der Platinmetall-katalysierten Anlagerung SiH-Gruppen tragender Siloxane und Silane an olefinisch funktionalisierte Verbindungen, wie z.B. an Allylpolyether.
Die Verwendung von Platinkatalysatoren für die Anlagerung von Silanen oder Siloxanen mit SiH-Gruppen an Verbindungen mit einer oder mehreren olefinischen Doppelbindungen ist bekannt (Hydrosilylierung) und z.B. in dem von Michael. A. Brook verfassten Buch "Silicon in Organic, Organometallic, and Polymer Chemistry", Verlag John Wiley & Sons, Inc., New York 2000, Seite 403 ff., und in der Patentliteratur, z.B. in der DE-A-26 46 726, der EP-A-0 075 703 und der US-A-3 775 452, beschrieben. In der heutigen betrieblichen Praxis haben sich überwiegend Hexachloroplatinsäure und cis-Diamminoplatin(II)chlorid durchgesetzt.

So einfach sich dieses Reaktionsprinzip beschreiben lässt, so kompliziert gestaltet sich oft dessen reproduzierbare Durchführung im industriellen Maßstab.

Einmal verläuft diese Anlagerungsreaktion nur dann ohne nennenswerte Bildung von Nebenprodukten, wenn die Verbindungen, welche olefinische Doppelbindungen aufweisen, frei von Gruppen sind, die in Konkurrenz zur Anlagerungsreaktion mit der SiH-Gruppe reagieren können. Hierzu ist insbesondere die an Kohlenstoff gebundene Hydroxylgruppe zu rechnen.

Zum anderen sind die erforderlichen Platinmetallmengen (ausgedrückt in Form von Gewichtsteilen pro Million Gewichtsteile der jeweils betrachteten Hydrosilylierungsmischung) um zu brauchbaren Ergebnissen zu kommen, häufig derart hoch, dass diese Verfahren wirtschaftlich uninteressant werden.

Insbesondere führen jedoch mangelnde SiH-Umsätze zu unerwünschtem Molekulargewichtsaufbau durch Neuknüpfung von SiOSi-Bindungen. Als Folge dieser Quervemetzung kann beispielsweise die Viskosität dieser Produkte nicht in den spezifizierten Bereichen gehalten werden, aber auch deren angestrebte tensidische Wirkung kann erhebliche Einbußen erfahren.

Selbst aktive Katalysatorsysteme, wie z.B. diejenigen vom Karstedt-Typ (US 3 814 730), neigen bei der Herstellung organomodifizierter Siloxane, insbesondere der Allylpolyethersiloxane, zu Desaktivier- und Abschaltphänomenen, so dass sich oft die Notwendigkeit zur Nachkatalyse und/oder auch der drastischen Temperaturanhebung in der Anlagerungsreaktion ergibt.

Auch ist in manchen Fällen festgestellt worden, dass es für die Hydrosilylierungsprodukte abträglich ist, wenn die bekannten Platinkatalysatoren oberhalb der normalen Katalysatorgewichtsanteile und/oder bei hohen Temperaturen verwendet werden. Durch diese verschärften Reaktionsbedingungen wird die Bildung umgelagerter Nebenprodukte forciert und die Desaktivierung des Katalysators z.B. durch irreversible Ausfällung von Edelmetall aus der Reaktionsmatrix gefördert.

Die anwendungstechnische Nutzbarkeit von Produkten, die aus der Platinmetall-katalysierten Anlagerungsreaktion von SiH-Gruppen tragenden Siloxanen an olefinische Doppelbindungen aufweisende Verbindungen hervorgehen, ist insbesondere direkt verknüpft mit dem erzielten Umsatz in der Hydrosilylierung; d.h., der Minimierung von restlichen SiH-Funktionen. Rest-SiH führt insbesondere in Gegenwart ubiquitärer Wasserspuren (z.B. Luftfeuchtigkeit) zu unkontrollierbaren Hydrolyse- und Vemetzungsprozessen, die speziell im Falle von Anlagerungsverbindungen hohen Molekulargewichts zwingend zur Vergelung führen und die Produkte unbrauchbar machen.

Es hat in der Praxis nicht an Anstrengungen gefehlt, gerade auch im Falle der als Emulgatoren eingesetzten Alkylsiloxan-Polyethersiloxan-Copolymeren, die im Rahmen einer mehrstufigen Anlagerungsreaktion hergestellt werden, restliche SiH-Funktionen durch eine Beaufschlagung der Reaktionsmatrix mit überschüssigem Ethylen als SiH-bindendes Hilfsolefin abzufangen. Diese Maßnahme besitzt jedoch nicht die gewünschte Effizienz, so dass ca. 2 bis 3 % nichtumgesetzten Siliciumwasserstoffs (bezogen auf das Ausgangssiloxan) verbleiben. Erfahrungsgemäß ist ein derartiges Produkt nicht lagerstabil und erleidet Vergelung.

In diesem Zusammenhang erweisen sich als besonders empfindliche Indikatoren für Abweichungen vom Qualitätsniveau auch z.B. diejenigen Polyethersiloxane, die als Schaumstabilisatoren Eingang nehmen in die Herstellung von PU-Weichschäumen. Der Hauptweg zu dieser technisch bedeutsamen Verbindungsklasse führt über die Edelmetall-katalysierte Anlagerung von Allylalkohol gestarteten Polyoxyalkylenverbindungen (Allylpolyether) an Poly(methylwasserstoff)-polydimethylsiloxan- Copolymere. Als anwendungstechnische Parameter sind sowohl die Aktivität als auch die Zellfeinheit Kriterien zur Beurteilung der Stabilisatorqualität. Verfahrensänderungen in der Stabilisatorherstellung, wie z.B. die Änderung der Katalysebedingungen während der SiC-Verknüpfungsreaktion, haben unmittelbar Einfluss auf die Schaumqualität.

Die Edelmetall-katalysierte Hydrosilylierungsreaktion erschließt durch die Vielzahl an Kombinationsmöglichkeiten zwischen SiH-Gruppen enthaltenden Silanen bzw. Siloxanen und olefinisch ungesättigten Verbindungen ein breites Spektrum modifizierter Silane bzw. Siloxane.

Die Eignung von Di-µ-chloro-bis(1,2-η)-cyclohexen platin(II)chlorid zur Herstellung von organomodifizierten Polysiloxanen gelöst in ionischen Flüssigkeiten ist in der EP 1 382 630 beschrieben. Im Beispiel 9 der zitierten Schrift wird ein kurzkettiges, hochreaktives α,ω-SiH-Polydimethylsiloxan der Kettenlänge N= 20 mit 1,3 Äquivalenten eines ungesättigten Polyethers einer mittleren Molmasse von 400 g/ mol und einem Ethylenglykolanteil von 100% auf 90°C erhitzt. Dieser Reaktionsmischung werden 20 ppm Di-µ-chloro-bis(1,2-η)-cyclohexen platin(II)chlorid gelöst in 1,2,3-Trimethylimidazolium- methylsulfat hinzugefügt und der gesamte Reaktionsansatz wird für 5 Stunden bei 90°C gerührt. Nach Abkühlen des Reaktionsansatzes gestattet eine Phasenseparation die Abtrennung des ABA-strukturierten Polyethersiloxans von der den Platinkomplex enthaltenden 1,2,3-Trimethylimidazoliummethylsulfat-Phase. Die EP 1 382 630 zeigt nicht, dass sich Di-µ-chloro-bis(1,2-η)-cyclohexen platin(II)chlorid ohne Verwendung von ionischen Flüssigkeiten als Hilfsphase als Katalysator für die Herstellung organomodifizierter Polysiloxane eignet. Bei existierenden Anlagen zur Herstellung organomodifizierter Siloxane sieht man üblicherweise keine technischen Einrichtungen zur Phasenseparation und zur Kreislaufführung von Hilfsphasen vor, so dass die hier diskutierte technische Lehre nur mit erheblichem Aufwand und Kapitaleinsatz umzusetzen ist.

In der EP 0 073 556 wurde Di-µ-chloro-bis(1,2-η)-cyclohexen platin(II)chlorid zusammen mit einer aliphatisch ungesättigten Organosiloxanverbindung und einem Aluminiumalkoxid zu einem SiC-Verknüpfungen auslösenden Katalysatorsystem kombiniert, wobei die Zielstellung dieses Schutzrechtsersuchens die beschleunigte Aushärtung von Release-Coatings auf Basis von Vinylsiloxan/ SiH-Siloxan-Systemen ist. Die Verknüpfungsreaktion erfolgt in einer reinen Siloxanphase. Aufgabe ist es hier nicht organomodifizierte Siloxane - insbesondere nicht solche, die der Klasse der Polyethersiloxane angehören, via SiC-Verknüpfung herzustellen. Für die Gewinnung von Siloxanderivaten tensidischer Natur, bei denen die Polaritätsdifferenz zwischen den im Wesentlichen unpolaren Siloxanen und den deutlich hydrophileren Additionspartnern oft eine Reaktionsführung aus der vom Reaktionsbeginn gesehen initialen Zweiphasigkeit bedingt, weist die Lehre der EP 0 073 556 somit nicht den Lösungsweg.

Die DE-OS-1793494 stellt auf Katalysatorzusammensetzungen aus Platin für Hydrosilylierungsreaktionen ab, wobei olefinische Chlorokomplexe des Platins mit cyclischen Alkylvinylpolysiloxanen unter Substitution des ursprünglich am Platin gebundenen Olefins zur Umsetzung gebracht werden. Die so erhaltenen Alkenylsiloxanplatinhalogenid-Komplexe werden für die Anlagerung eines SiH-Gruppen tragenden Organopolysiloxans an ein weiteres, aliphatisch ungesättigte Gruppen aufweisendes Organopolysiloxan benutzt, da sie der dort vertretenen erfinderischen Lehre gemäß, eine erhöhte Systemlöslichkeit gegenüber den als Katalysatoredukt eingesetzten olefinischen Chlororokomplexen des Platins besitzen.

Mit der US 3,159,601 hatte Ashby bereits die Verwendung von rein olefinischen Chlorokomplexen des Platins für die Anlagerung eines SiH-Gruppen tragenden Organopolysiloxans an ein weiteres, aliphatisch ungesättigte Gruppen aufweisendes Organopolysiloxan reklamiert. Weder die DE-OS-1793494 noch die US 3,159,601 zeigen die Tauglichkeit von olefinischen Platinchlorokomplexen für die SiC-verknüpfende Herstellung organomodifizierter Siloxane.

Die DE 1210844 beansprucht die Anlagerung von Silanen an ungesättigte Kohlenwasserstoffe unter Bildung von siliciumhaltigen Kohlenwasserstoffen. Dabei ist der Katalysator Di-µ-chloro-bis(1,2-η)-cyclohexen platin(II)chlorid als Vergleichsverbindung benannt, der bei der Anlagerung von Methyldichlorsilan an Allylacetat zu einem schwarz verfärbten Produkt führt, was auf die Zersetzung des Katalysators selbst hinweist.

Aufgabe der vorliegenden Erfindung ist die Bereitstellung eines alternativen Katalysatorsystems bei der Anlagerung von SiH-Gruppen-haltigen Siloxanen an olefinische Doppelbindungen.

Überraschenderweise wurde nun gefunden, dass abseits des hier dargelegten Standes der Technik die Anlagerung SiH-Gruppen aufweisender Siloxane an olefinische Doppelbindungen tragende organische Verbindungen durch Verwendung von Di-µ-chloro-bis(1,2-η)cyclohexen platin(II)chlorid als Katalysator gelingt.
Besonders überraschend und für den Fachmann keinesfalls zu erwarten gelingt die durch Verwendung von Di-µ-chloro-bis(1,2-η)-cyclohexen platin(II)chlorid als Katalysator hervorgebrachte SiC-Verknüpfungsreaktion sogar aus der allein durch die Reaktionspartner definierten Matrix heraus; d.h., unter Verzicht auf zusätzliche Lösungsmittel oder weitere gegebenenfalls den Katalysator kompatibilisierende oder lösende Hilfsphasen.
Unter Kompatibilisierung ist in diesem Zusammenhang die Möglichkeit der homogenen Verteilung des Katalysators in der Reaktionsmatrix zu verstehen, ohne dass zusätzliche Lösemittel und/ oder Dispergiermittel für den Katalysator verwendet werden müssten. Der Katalysator kann überraschenderweise seine Wirkung auch ohne die Anwesenheit eines den Katalysator lösenden oder suspendierend/emulgierend wirkenden Zusatzes von Hilfsphasen entfalten.

Gegenstand der Erfindung ist daher ein Verfahren zur Herstellung von Umsetzungsprodukten aus SiH-Gruppen aufweisenden Siloxanen und olefinische Doppelbindungen tragenden organischen Verbindungen durch Verwendung von Di-µ-chloro-bis(1,2-η)-cyclohexen platin(II)chlorid als Katalysator.

Der Katalysator Di-µ-chloro-bis(1,2-η)cyclohexen platin(II)chlorid liegt dabei in den Reaktionskomponenten hauptsächlich in suspendierter Form vor. Die Reaktion findet somit in Masse statt.
Die Reaktionspartner, d.h. die SiH-Gruppen aufweisenden Siloxane sowie die olefinische Doppelbindungen aufweisenden organischen Verbindungen und Verfahren zu deren Herstellung sind bekannt. Die Archetypen der SiH-Gruppen aufweisenden Siloxane sind z. B. in dem von Walter Noll verfassten Standardwerk "Chemie und Technologie der Silicone", Verlag Chemie GmbH, Weinheim/Bergstrasse (1960), eingehend beschrieben.

Die SiH-Gruppen in den Siloxanen können terminal und/oder nicht-terminal sein. Erfindungsgemäß verwendbare Siloxane sind Verbindungen der allgemeinen Formel (I) 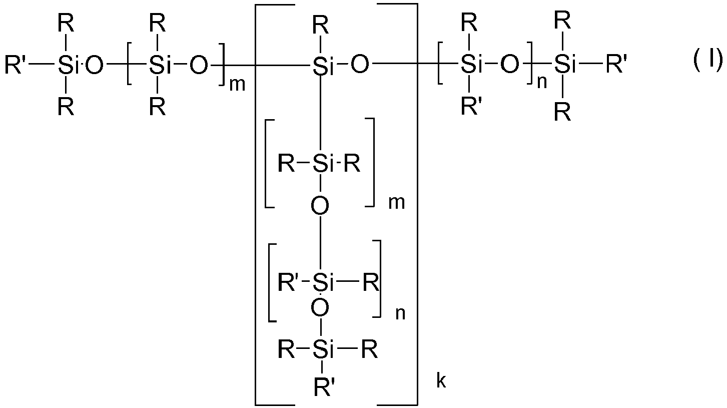 worin
- R: ein substituierter oder unsubstituierter Kohlenwasserstoffrest mit 1 bis zu 20 C-Atomen sein kann, vorzugsweise eine Methylgruppe ist,
- R': Wasserstoff und/oder R sein kann,
- m: 0 bis 500, vorzugsweise 10 bis 200, insbesondere 15 bis 100,
- n: 0 bis 60, vorzugsweise 0 bis 30, insbesondere 0,1 bis 25,
- k: 0 bis 10, vorzugsweise 0 bis 4, ist,
mit der Maßgabe, dass R' mindestens einmal Wasserstoff ist.

Die Siloxane sind technische Produkte, in denen die einzelnen Bestandteile der in der allgemeinen Formel (I) in Klammem aufgeführten Teile statistisch verteilt als auch blockweise enthalten sein können, sie können, durch die Herstellung bedingt, auch größere Anteile an Verzweigungen enthalten. Die erfindungsgemäß bevorzugten Verbindungen sind im Wesentlichen linear. In Anteilen von 50 Gew.-%, vorzugsweise > Gew.-90 % sind die Reste R kurzkettige Alkylreste, insbesondere Methylreste.

Bevorzugt ist als Rest R eine oder mehrere gleiche oder verschiedene Gruppen, die die Anlagerungsreaktion nicht behindern, wie Alkylgruppen mit 1 bis 8 Kohlenstoffatomen; substituierte Alkylgruppen mit 1 bis 8 Kohlenstoffatomen, wie die 3-Chlorpropyl-, 1-Chlormethyl-, 3-Cyanopropylgruppe; Arylgruppen, wie die Phenylgruppe; Aralkylgruppen mit 7 bis 20 Kohlenstoffatomen, wie die Benzylgruppe; Alkoxy- oder Alkoxyalkylgruppen, wie die Ethoxy- oder Ethoxypropylgruppe. Dabei kann innerhalb eines Moleküls der Formel (I) der Rest R auch verschiedene Bedeutungen haben. Bevorzugt sind jedoch Verbindungen, bei denen alle Reste R oder deren überwiegende Zahl die Bedeutung eines Methylrestes haben.

Beispiele geeigneter bevorzugter Siloxane mit SiH-Gruppen sind Verbindungen der Formel (II) oder (III): 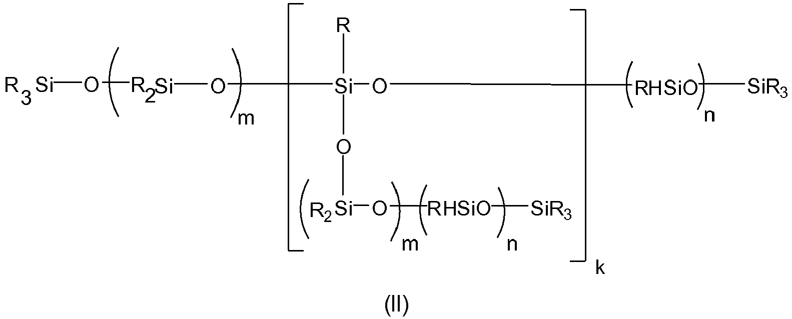 wobei die Indices und Reste die gleichen Definitionen besitzen wie unter Formel (I) beschrieben; 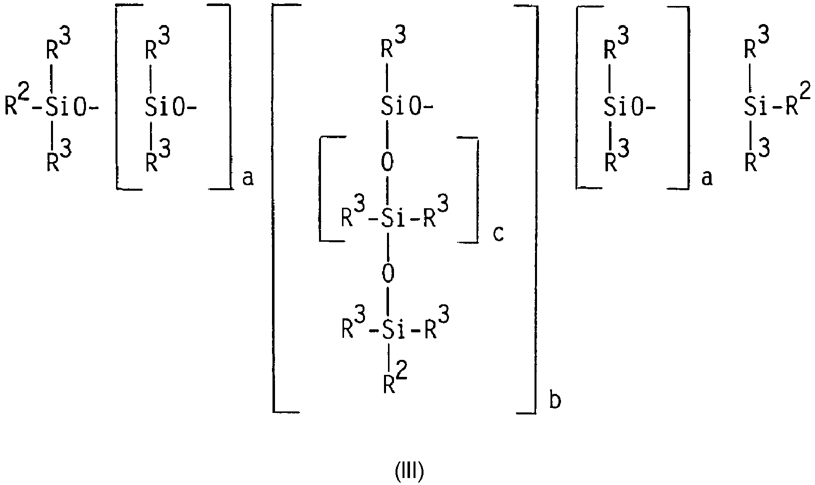 wobei
- R³: im durchschnittlichen Molekül Alkylreste mit 1 bis 18 Kohlenstoffatomen oder Arylreste bedeuten, jedoch mindestens 90% der Reste R Methylreste sind,
- R²: die Bedeutung der Reste R³ hat oder Wasserstoffreste sind, wobei im durchschnittlichen Molekül mindestens 2 Reste R² Wasserstoffreste sein müssen,
- a: einen Wert von 0,5 bis 100,
- b: einen Wert von 0 bis 5 und
- c: einen Wert von 0 bis 100 hat.

Besonders bevorzugt sind Wasserstoffpolysiloxane, bei denen R² Wasserstoffreste und R³ Methylreste sind, a einen Wert von 0,5 bis 5, b den Wert 0 hat und c einen Wert von 1 bis 10 hat.

Erfindungsgemäß sind die olefinisch ungesättigten organischen Verbindungen ausgewählt aus der Gruppe der α-Olefine, der gespannten Ringolefine, der α,ω-Alkenole, der terminal olefinisch ungesättigten Polyether, der aminofunktionellen α-Olefine oder der α-Olefingruppen tragenden Oxirane sowie aus der Gruppe der in ω-Position olefinisch ungesättigten Carbonsäureester bzw. auch aus Mischsystemen der hier aufgeführten Substanzklassen.

Bei den α-Olefinen handelt es sich um verzweigte oder unverzweigte α-Olefine mit 2-18 Kohlenstoffatomen, die einfach oder mehrfach ungesättigt sind, bevorzugt sind Ethylen, Propen-1, Buten-1, iso-Buten, besonders bevorzugt sind Hexen, Octen, Decen, Undecen, Hexadecen, Octadecen sowie α-Olefine im C-Zahlbereich von C₂₀-C₄₀. sowie die technisch allgemein verfügbaren C₂₂-C₂₄-Olefin-Schnitte aus der Petrochemie.

Die olefinisch ungesättigten Verbindungen können jeweils allein oder in beliebigen Mischungen mit weiteren olefinisch ungesättigten Verbindungen eingesetzt werden. Werden Mischungen verwendet, so entstehen bei der Umsetzung blockartig oder statisch aufgebaute Copolymerverbindungen, je nachdem ob die ungesättigten Verbindungen gleichzeitig oder zeitlich versetzt oder abwechselnd zum SiH-Siloxan dosiert werden. Dem Fachmann sind die Auswahlkriterien bekannt unter denen er die Olefine auszuwählen hat um zu besonders vorteilhaften Produkteigenschaften zu gelangen. Insbesondere bei Mischungen unterschiedlicher Olefine weiß der Fachmann Nebenreaktionen unterschiedlicher funktioneller Gruppen der Mischungskomponenten einzuschätzen und gegebenenfalls zu unterbinden. So wird beispielsweise die Mischung eines aminofunkionellen Olefins mit einem olefingruppentragenden Oxiran zu der unvermeidbaren Nebenreaktion von Aminogruppe und Oxiran-Ring unter Ringöffnung führen.

Bei den gespannten Ringolefinen sind namentlich die Derivate des Norbornens und des Norbomadiens, des Dicyclopentadiens sowie deren unsubstituierten Grundkörper zu nennen.

Bei den α,ω-Alkenolen handelt es sich um verzweigte oder unverzweigte α,ω-Alkenole mit 2-18 Kohlenstoffatomen die einfach ungesättigt sind. Bevorzugt für die Klasse der α,ω-Alkenole seien 5-Hexen-1-ol und 9-Decen-1-ol genannt.

Unter terminal olefinisch ungesättigten Polyethern werden solche Polyoxyalkylenverbindungen verstanden, deren ungesättigter Terminus sich durch eine Vinyl-, Allyl- oder Methallylgruppe definiert.

Erfindungsgemäß dafür sind Polyoxyalkylenverbindungen der Formeln

CH₂=CH-CH₂-O-(CH₂-CH₂-O-)ₓ-(CH₂-CH(R')O-)_{y}(SO)_{z}-R" (IV)

CH2=CH-O-(CH2-CH2-O-)ₓ-(CH2-CH(R')O-)_{y}-R" (V)

CH₂=CH-CH₂-R^{IV} (VI)

CH₂=CH-(O)_{x'}-R^{IV} (VII)

worin
- x: = 0 bis 100,
- x': = 0 oder 1,
- y: = 0 bis 100,
- z: = 0 bis 100,
- R': eine gegebenenfalls substituierte Alkylgruppe mit 1 bis 4 C-Atomen ist und
- R": einen Wasserstoffrest oder eine Alkylgruppe mit 1 bis 4 C-Atomen; die Gruppe -C(O)-R'" mit R'" = Alkylrest;
die Gruppe -CH₂-O-R'; eine Alkylarylgruppe, wie die Benzylgruppe; die Gruppe -C(O)NH-R' bedeutet,
- R^{IV}: ein gegebenenfalls substituierter Kohlenwasserstoffrest mit 7 bis 47, vorzugsweise 13 bis 37 C-Atomen,
- SO: der Rest C₆H₅-CH(-)-CH₂-O-
bedeutet.

Unter den aminofunktionellen α-Olefinen werden hier insbesondere Allylamin und N-Ethylmethallylamin verstanden.

Ryang weist in der Lehre der US 4,892,918 Hexachloroplatinsäure als denjenigen Hydrosilylierungskatalysator aus, derfür die Herstellung sekundärer aminofunktioneller Siloxane am geeignetsten ist. Überraschenderweise gelingt diese Synthese aber auch mit dem hier vorgestellten Katalysator Di-µ-chloro-bis(1,2-η)-cyclohexen platin(II)chlorid. Zwar wird in US 4,892,918 ebenfalls die Reaktion in Masse - d.h. ohne Anwesenheit von Lösungsmitteln oder anderen Hilfsphasen durchgeführt, aber die Reaktion der aminofunktionellen Siloxane legt in Kenntnis der hier vorgestellten erfinderischen Idee die Vermutung nahe, dass die Hexachloroplatinsäure durch die Aminofunktionen in eine teilweise gelöste Form überführt wird, die dann vergleichbar dem Di-µ-chloro-bis(1,2-η)-cyclohexen platin(II)chlorid zu einem wirksamen Katalysatorsystem führt. Allerdings zeigen die Beispiele der US 4,892,918, dass lange Reaktionszeiten in Kauf genommen werden müssen.

Überraschenderweise gelingt eine solche Reaktion mit dem erfindungsgegenständliche Verfahren und mit dem Katalysator Di-µ-chloro-bis(1,2-η)-cyclohexen platin(II)chlorid sowohl mit den aminfunktionellen Edukten, als auch ohne Anwesenheit von inhärent in der Molekülstruktur anwesenden und als Hilfsphasenvermittler wirkenden Gruppen. Selbst bei hohen Reaktionstemperaturen und unter Gewährleistung von vergleichsweise kurzen Reaktionszeiten bei hohen Umsatzraten werden im erfindungsgegenständlichen Verfahren nahezu farblose Reaktionsprodukte erhalten (siehe Beispiele 5 und 7 für aminofunktionelle Substrate und die weiteren erfindungsgemäßen Beispiele).

Als Stellvertreter, jedoch keinesfalls limitierend für diese Stoffgruppe der mit anlagerungsfähigen olefinischen Funktionen versehenen Oxirane seien hier Allylglycidylether und Vinylcyclohexenoxid genannt.

Unter in ω-Position olefinisch ungesättigten Carbonsäureestem firmieren solche wie beispielsweise der technisch gut zugängliche Undecylensäuremethylester.

Die Hydrosilylierungsreaktion wird vorzugsweise mit einem gewissen, mindestens etwa 15 mol%igem Überschuss von Alkenen, bezogen auf eine SiH-Gruppe, vorgenommen. Im Falle ungesättigter Polyether, insbesondere Allylpolyether als Reaktanten werden technisch übliche Überschüsse von circa 30-40 Mol-% gewählt. Lösungsmittel müssen nicht eingesetzt werden, stören jedoch nicht, falls sie, in Bezug auf die Reaktion, inert sind. Die Reaktionstemperatur liegt im Allgemeinen und vorzugsweise bei etwa 140° C bis 160° C. Die Reaktionsdauer beträgt 1 bis 8, vorzugsweise 1 bis 3 Stunden.

Die einzusetzende Menge an Platinkatalysator Di-µ-chloro-bis(1,2-η)cyclohexen platin(II)chlorid richtet sich im Wesentlichen nach der Reaktivität und dem Molekulargewicht der Reaktionspartner. Im Allgemeinen verwendet man 10⁻² bis 10⁻⁸ Mol, vorzugsweise 10⁻³ bis 10⁻⁶ Mol des Katalysators auf jeweils 1 Mol SiH-Gruppen im Siloxan.

Der erfindungsgemäße Katalysator kann über einen weiten Temperaturbereich eingesetzt werden. Zur Vermeidung der im Stand der Technik dargelegten produktschädigenden Nebenreaktionen wird vorzugsweise der Temperaturbereich so niedrig gewählt, dass er einen akzeptablen Kompromiss zwischen angestrebter Produktreinheit und Produktionsleistung darstellt. Zur Erzielung höherer Durchsatzraten kann die Umsetzungstemperatur auch erheblich erhöht werden (bis ca. 150 °C), ohne dass es zu Desaktivier- und Abschaltphänomenen kommt.

Die erfindungsgemäss erhaltenen Aminogruppen aufweisenden linearen Polydimethylsiloxane können zur Behandlung von Textilien verwendet werden, um ihnen einen weichen Griff und eine gewisse Antistatik zu vermitteln.

Ferner gestatten die nach dem erfindungsgemäßen Verfahren erhaltenen Aminogruppen aufweisenden linearen Polydimethylsiloxane durch deren weitere Umsetzung mit z.B. α,ω-Epoxidfunktionen tragenden Polypropylenoxiden und Diaminen wie z.B. Piperazin den Aufbau Weichgriff verbessernder Waschflottenadditive, wie in der bisher noch nicht veröffentlichten Patentanmeldung DE 10 2010 001350.1 ausgeführt wird.

Die erhaltenen Verbindungen können aber insbesondere als reaktive Komponenten zur Herstellung polymerer Verbindungen eingesetzt werden:
Eine Verwendungsmöglichkeit besteht darin, sie als Vemetzungskomponente in Epoxidharzen zur Verbesserung der Zähigkeit der Epoxidharze, insbesondere bei tiefen Temperaturen von unter 0 °C, einzusetzen.

Ein weiterer Verwendungszweck ist ihre Umsetzung mit Siloxanen, welche endständig Epoxidgruppen aufweisen, um Polymere zu erhalten, welche zur Beschichtung von Textilien eingesetzt werden. Diese Beschichtungen verleihen den Textilien einen weichen Griff.

Üblicherweise werden die erfindungsgemäß beanspruchten Reaktionen unter atmosphärischem Druck, gegebenenfalls jedoch auch unter erhöhtem Druck durchgeführt.

Erfindungsgemäß bevorzugt wird das Verfahren bei Normaldruck durchgeführt, davon abweichende Druckbereiche sind -falls erwünscht-jedoch ebenfalls möglich.

Die erfindungsgemäß hergestellten Organosiloxane können anstelle der für alle jeweiligen Anwendungszwecke in Haushalt und Industrie sowie in Reinigungs- und Pflegemitteln für Haut und Hautanhangsgebilde, in Reinigungs- und Pflegeformulierungen für die Anwendung in Pharmazie, Haushalt und Industrie verwendeten aber konventionell hergestellten organomodifizierten Organosiloxane und der darauf basierenden wässrigen Systeme verwendet werden. Auf Grund der überaus vorteilhaften rheologischen Eigenschaften sind sie darüber hinaus auch für bislang unzugängliche Anwendungsgebiete verwendbar.

Beispielsweise aber nicht abschließend sind dies Pigmentnetzmittel oder Dispergieradditive zur Herstellung von homogenen, lagerstabilen Pasten, Farben, Lacken, Überzügen, Beschichtungen, Anstrichmitteln; in Antitranspirantien/ Deodorantien, sowie in pharmazeutischen Formulierungen.

Ein weiterer Gegenstand ist der Einsatz der erfindungsgemäß hergestellten Organosiloxane in Mitteln zur Reinigung und Pflege harter Oberflächen, sowie zur Ausrüstung, Reinigung und Pflege von Textilien.

Ein weiterer Gegenstand ist die Verwendung der verfahrensgemäß hergestellten organomodifizierten Organosiloxane bei der Behandlung und Nachbehandlung von Textilien, z. B. als Reinigungs- und Pflegemittel, als Imprägniermittel, Avivagehilfsmittel und Griffverbesserer und Textilweichmacher.

Ein weiterer Gegenstand ist die Verwendung der erfindungsgemäß hergestellten Organosiloxane, insbesondere der Siliconpolyethercopolymere bei der Herstellung von Polyurethanschäumen, z.B. als Schaumstabilisatoren, Zellöffner, Trennmittel, etc.

Das Verfahren wird im Allgemeinen so durchgeführt, dass die SiH-Verbindungen a) zumindest teilweise, d.h. weitgehend, vorzugsweise aber möglichst vollständig mit der Doppelbindung der Komponente b) umgesetzt werden.

Ein weiterer Gegenstand ist die Verwendung der organisch modifizierten Siloxane bei der Herstellung von Polyestern und Polyurethanen. Insbesondere können die organisch modifizierten Siloxane enthaltend Aminofunktionen bei der Herstellung von Polyestern und Polyurethanen als Weichsegment im Molekül fungieren.

Weitere Gegenstände der Erfindung sind in den Ansprüchen beschrieben, deren Offenbarungsgehalt vollumfänglich Bestandteil dieser Beschreibung ist.

In den nachfolgend aufgeführten Beispielen wird die vorliegende Erfindung zur Verdeutlichung der Erfindung beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Sind in dieser Beschreibung oder den Beispielen Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z.B. organomodifizierte Siloxane beschrieben, die verschiedene Monomereinheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als statistische Mittelwerte, gemittelt über alle entsprechenden Verbindungen, zu verstehen.

### Experimenteller Teil:

Die hier aufgeführten Beispiele dienen der Illustration des erfindungsgemäß beanspruchten Verfahrens.

Die Bestimmungen der SiH-Werte der eingesetzten Wasserstoffsiloxane aber auch die der Reaktionsmatrices erfolgen jeweils gasvolumetrisch durch die Natriumbutylat induzierte Zersetzung aliquot eingewogener Probemengen an einer Gasbürette. Eingesetzt in die allgemeine Gasgleichung gestatten die gemessenen Wasserstoffvolumina die Gehaltsbestimmung aktiver SiH-Funktionen in den Edukten aber auch in den Reaktionsansätzen und erlauben so die Umsatzkontrolle.

Die Bestimmung der Molekulargewichte der eingesetzten Allylalkohol gestarteten Polyether (Beispiele 1 und 6) aber auch des technischen α-Olefins (Beispiel 2) erfolgte durch die Titration der Jodzahl nach Hanus, die seit langem Bestandteil der Abteilung C-V der "DGF-Einheitsmethoden" ist (vergleiche Fat Sci. Technol. 1991, Nr. 1, Seiten 13-19 sowie DGF C-V 11 a (53)und Ph. Eur. 2.5.4 (Methode A).

Der erfindungsgemäß eingesetzte Katalysator kann über W.C. Heraeus, Hanau, DE kommerziell erworben werden.

Die angegeben Viskositäten werden durch Messung in einem Viskosimeter der Fa. Haake als dynamische Scherviskositäten gemäß DIN 53921 bestimmt.

### Beispiel 1:

### Herstellung eines Siliconpolyethercopolymers:

In einem 1-1-Mehrhalsnindkolben mit Innenthermometer, KPG-Rührer und Rückflußkühler werden 410 g eines Allylalkohol gestarteten Polyethers mit einem mittleren Molekulargewicht von ca. 1840 g/ mol und ca. 80% Propylenoxidanteil und einer Jodzahl von 13,8 gemeinsam mit 130 g eines Polydimethylsiloxan-Polymethylwasserstoffsiloxan-Copolymeren des mittleren Molekulargewichts 5100 g/ mol und einem SiH-Wert von 1,27 mol/ kg unter Rühren auf 100°C erhitzt. 24 mg Di-µ-chloro-bis(1,2-η)-cyclohexen platin(II)chlorid werden hinzugefügt und der Reaktionsansatz wird für weitere 3 Stunden bei 100°C gehalten.
Eine zur gasvolumetrischen SiH-Bestimmung (Bestimmung des bei der Zersetzung einer aliquoten Menge mit Hilfe einer Natriumbutylatlösung entwickelten Wasserstoffs an einer Gasbürette) entnommene Probe sichert quantitativen SiH-Umsatz. Erhalten wird nach Abkühlung ein nahezu farbloses Siliconpolyether-Copolymer mit einer Viskosität von 920 mPas.

Überraschenderweise gelingt die Umsetzung bei vergleichsweise hoher Temperatur sehr schnell und insbesondere ohne Bildung von (gefärbten) Nebenprodukten. Eine aufwändige Aufreinigung des Produkts kann daher entfallen.

### Beispiel 2:

### Herstellung eines Siliconwachses:

In einem 1-1-Mehrhalsrundkolben mit Innenthermometer, aufgesetztem Tropftrichter, KPG-Rührer und Rückflußkühler werden 316 g eines α-Olefins mit einem mittleren Molekulargewicht von ca. 350 g/ mol unter Rühren auf 130°C erwärmt und mit 17 mg Di-µ-chloro-bis(1,2-η)-cyclohexen platin(II)chlorid versetzt. 50 g eines Polymethylwasserstoff-siloxans mit einem SiH-Gehalt von 15,7 mol/kg werden aus dem Tropftrichter im Verlauf von 1,5 Stunden hinzugegeben Die von starker Exothermie gekennzeichnete Reaktion ist nach 2 Stunden beendet. Die gasvolumetrische SiH-Bestimmung (Bestimmung des bei der Zersetzung einer aliquoten Menge mit Hilfe einer Natriumbutylatlösung entwickelten Wasserstoffs an einer Gasbürette) belegt vollständigen Umsatz. Man isoliert nach Abkühlen ein farbloses Siliconwachs mit einem Schmelzpunkt von 68°C.

### Beispiel 3:

### Herstellung eines Alkenol-Silicon-Anlagerungsproduktes:

In einem 500-ml-Mehrhalsrundkolben mit Innenthermometer, aufgesetztem Tropftrichter, KPG-Rührer und Rückflußkühler werden 164,5 g 5-Hexen-1-ol mit unter starkem Rühren auf 85°C erwärmt und mit 18 mg Di-µ-chloro-bis(1,2-η)-cyclohexen platin(II)chlorid versetzt. 230 g eines αω-Dihydrogenpolydimethylsiloxan-Polymethylwasserstoffsiloxan-Copolymerens mit einem SiH-Gehalt von 5,5 mol/kg werden aus dem Tropftrichter in einer solchen Geschwindigkeit hinzugegeben, dass die Reaktionstemperatur nicht über 120°C steigt. Die stark exotherme Reaktion ist nach 3 Stunden beendet. Die gasvolumetrische SiH-Bestimmung (Bestimmung des bei der Zersetzung einer aliquoten Menge mit Hilfe einer Natriumbutylatlösung entwickelten Wasserstoffs an einer Gasbürette) belegt vollständigen Umsatz. Man isoliert nach Abkühlen ein farbloses Alkenol-Silicon-Anlagerungsprodukt mit einer Viskosität von 2100 mPas.

### Beispiel 4:

### Herstellung eines Undecylensäuremethylester-modifizierten Siliconwachses:

In einem 500-ml-Mehrhalsrundkolben mit Innenthermometer, aufgesetztem Tropftrichter, KPG-Rührer und Rückflußkühler werden 190,0 g eines Polydimethylsiloxan-Polymethylwasserstoffsiloxan-Copolymeren mit einem SiH-Gehalt von 3,54 mol/ kg unter Rühren auf 110°C erhitzt. und mit 21 mg Di-µ-chloro-bis(1,2-η)-cyclohexen platin(II)chlorid versetzt. Ein Gemisch bestehend aus 107 g Undecylensäuremethylester und 174 g Hexadecen wird aus dem Tropftrichter in einer solchen Geschwindigkeit hinzugegeben, dass die Reaktionstemperatur nicht über 120°C steigt. Die stark exotherme Reaktion ist nach 3,5 Stunden beendet. Die gasvolumetrische SiH-Bestimmung (Bestimmung des bei der Zersetzung einer aliquoten Menge mit Hilfe einer Natriumbutylatlösung entwickelten Wasserstoffs an einer Gasbürette) belegt vollständigen Umsatz. Man isoliert nach Abkühlen ein farbloses Copolymer mit einer Viskosität von 410 mPas

### Beispiel 5:

### Herstellung eines Amino-funktionalisierten Polydimethylsiloxans:

In einem 500-ml-Mehrhalsrundkolben mit Innenthermometer, aufgesetztem Tropftrichter, KPG-Rührer und Rückflußkühler werden 160,3 g eines α,ω-Dihydrogenpolydimethylsiloxans mit einem SiH-Gehalt von 3,00 mol/ kg unter kräftigem Rühren auf 145°C erhitzt. und mit 10 mg Di-µ-chloro-bis(1,2-η)-cyclohexen platin(II)chlorid versetzt. Über den Tropftrichter werden 62 g N-Ethylmethallylamin mit einer Geschwindigkeit zudosiert, dass die Reaktionstemperatur unter 160°C bleibt. Bereits 1 Stunde nach beendeter Dosage sichert eine gasvolumetrische SiH-Bestimmung an einer gezogenen Probe vollständigen Umsatz. Nach Austausch des Rückflußkühlers gegen eine Destillationsbrücke wird der Ansatz bei 150°C Sumpftemperatur und 10 mbar Druck von flüchtigen Bestandteilen befreit. Nach Abkühlen wird ein nahezu farbloses, aminofunktionelles Polydimethylsiloxan isoliert, das eine Viskosität von 10 mPas aufweist.

Überraschenderweise gelingt die Umsetzung bei vergleichsweise hoher Temperatur sehr schnell und insbesondere ohne Bildung von (gefärbten) Nebenprodukten. Gerade die häufig bei amino-funktionalisierten Siloxanen auftretenden vergelten, stark verfärbten Produkte werden nicht erhalten. Eine aufwändige Aufreinigung des Produkts kann daher entfallen.

### Beispiel 6:

### Herstellung eines Epoxy-modifizierten Siliconpolyethers:

In einem 500-ml-Mehrhalsrundkolben mit Innenthermometer, aufgesetztem Tropftrichter, KPG-Rührer und Rückflußkühler werden 200,0 g eines Polydimethylsiloxan-Polymethylwasserstoffsiloxan-Copolymeren mit einem SiH-Gehalt von 1,55 mol/ kg gemeinsam mit 125,0 g eines Polyalkylenols eines mittleren Molekulargewichts von ca. 500 g/ mol und ca. 50% Propylenoxidanteil und einer Jodzahl von 49 unter kräftigem Rühren und Zusatz von 0,05 % Natriumcarbonat auf 100°C erhitzt und dann mit 15 mg Di-µ-chloro-bis(1,2-η)-cyclohexen platin(II)chlorid versetzt. Nach Abklingen der von Exothermie gekennzeichneten SiC-Verknüpfungsreaktion werden 13,8 g Allylglycidylether langsam hinzugetropft. Bereits eine Stunde nach beendeter Zugabe ist der gasvolumetrisch bestimmte Reaktionsumsatz quantitativ. Der Ansatz wird nach Austausch des Rückflußkühlers gegen eine Destillationsbrücke bei 130°C und 10 mbar Druck von Flüchtigen befreit. Nach Filtration und Abkühlen erhält man ein nahezu farbloses Copolymer mit einer Viskosität von 740 mPas.

### Beispiel 7:

### Herstellung eines α,ω- Aminopropylgruppen tragenden Polydimethylsiloxans (erfindungsgemäß):

In einem 500-ml-Mehrhalsrundkolben mit Innenthermometer, aufgesetztem Tropftrichter, KPG-Rührer und Rückflußkühler werden 160,3 g eines α,ω-Dihydrogenpolydimethylsiloxans mit einem SiH-Gehalt von 3,00 mol/ kg unter kräftigem Rühren auf 145°C erhitzt. und mit 30 ppm Di-µ-chloro-bis(1,2-η)-cyclohexen platin(II)chlorid versetzt. Über den Tropftrichter werden 148,2 g Allylamin mit einer Geschwindigkeit zudosiert, dass die Reaktionstemperatur unter 160°C bleibt. Bereits 4 Stunden nach Dosagebeginn sichert eine gasvolumetrische SiH-Bestimmung an einer gezogenen Probe vollständigen Umsatz. Nach Austausch des Rückflußkühlers gegen eine Destillationsbrücke wird der Ansatz bei 150°C Sumpftemperatur und 10 mbar Druck von flüchtigen Bestandteilen befreit. Nach Abkühlen wird ein nahezu farbloses, aminofunktionelles Polydimethylsiloxan isoliert, das eine Viskosität von 14 mPas aufweist und gemäß ²⁹Si-NMR-Analyse zu ca. 75% aus α-Anlagerungsprodukt und zu ca. 25% aus β-Anlagerungsprodukt besteht.

### Beispiel 8:

### Herstellung eines α,ω-Aminopropylgruppen tragenden Polydimethylsiloxans (nicht erfindungsgemäß):

In Analogie zu Beispiel 7 werden in einem 500-ml-Mehrhalsrundkolben mit Innenthermometer, aufgesetztem Tropftrichter, KPG-Rührer und Rückflußkühler 160,3 g eines α,ω-Dihydrogenpolydimethylsiloxans mit einem SiH-Gehalt von 3,00 mol/ kg unter kräftigem Rühren auf 145°C erhitzt. und mit 30 ppm des Platin-Pyridin-Katalysators (Pt (Pyridin)(Ethylen)Cl₂) versetzt. Über den Tropftrichter werden 148,2 g Allylamin mit einer Geschwindigkeit zudosiert, dass die Reaktionstemperatur unter 160°C bleibt. Erst 32 Stunden nach Dosagebeginn ist durch eine gasvolumetrische SiH-Bestimmung an einer gezogenen Probe vollständiger Umsatz nachweisbar. Nach Austausch des Rückflußkühlers gegen eine Destillationsbrücke wird der Ansatz bei 150°C Sumpftemperatur und 10 mbar Druck von flüchtigen Bestandteilen befreit. Nach Abkühlen wird ein dunkelgelb-bräunliches, aminofunktionelles Polydimethylsiloxan isoliert, das eine Viskosität von 18 mPas aufweist. Das zugehörige ²⁹Si-NMR-Spektrum belegt eine Selektivität der SiC-Verknüpfung wie im Beispiel 7, das heißt ca. 75% α-Anlagerungsprodukt und ca. 25% β-Anlagerungsprodukt. Gegenüber dem erfindungsgemäßen Beispiel 7 wird bei diesem Beispiel der Qualitätsunterschied deutlich. Zwar ist die Isomerenverteilung im Produkt vergleichbar dem Beispiel 7, jedoch ist eine wesentlich längere Reaktionszeit zu beobachten.

## Patentansprüche

1. Verfahren zur Herstellung von Umsetzungsprodukten aus SiH-Gruppen aufweisenden Siloxanen und olefinische Doppelbindungen tragenden organischen Verbindungen, **dadurch gekennzeichnet, dass** Di-µ-chloro-bis(1,2-η)-cyclohexen platin(II)chlorid als Katalysator verwendet wird und dass eine Reaktion ohne Lösungsmittel und ohne den Katalysator kompatibilisierende oder lösende Hilfsphasen durchgeführt wird und die Reaktion in Masse stattfindet, wobei die olefinisch ungesättigten organischen Verbindungen ausgewählt sind aus der Gruppe der α-Olefine, der gespannten Ringolefine, der α,ω-Alkenole, der terminal olefinisch ungesättigten Polyether, der aminofunktionellen α-Olefine oder der α-Olefingruppen tragenden Oxirane, oder der in ω-Position olefinisch ungesättigten Carbonsäureester, wobei die Olefine allein oder in beliebigen Mischungen miteinander eingesetzt werden können,
wobei unter terminal olefinisch ungesättigten Polyethern solche Polyoxyalkylenverbindungen verstanden werden, deren ungesättigter Terminus sich durch eine Vinyl-, Allyl- oder Methallylgruppe definiert,
ausgewählt aus Polyoxyalkylenverbindungen der Formeln
CH₂=CH-CH₂-O-(CH₂-CH₂O-)ₓ-(CH₂-CH(R')O-)_{y} (SO)_{z}-R" (IV)
CH₂=CH-O-(CH₂-CH₂O-)ₓ-(CH₂-CH(R')O-)_{y}-R" (V)
CH₂=CH-CH₂-R^{IV} (VI)
CH₂=CH-(O)_{x'}-R^{IV} (VII)
worin
x = 0 bis 100,
x' = 0 oder 1,
y = 0 bis 100,
z = 0 bis 100,
R' eine gegebenenfalls substituierte Alkylgruppe mit 1 bis 4 C-Atomen ist und
R" einen Wasserstoffrest oder eine Alkylgruppe mit 1 bis 4 C-Atomen; die Gruppe -C(O)-R'" mit R"' = Alkylrest; die Gruppe -CH₂-O-R'; eine Alkylarylgruppe, wie die Benzylgruppe; die Gruppe -C(O)NH-R' bedeutet,
R^{IV} ein gegebenenfalls substituierter Kohlenwasserstoffrest mit 7 bis 47, vorzugsweise 13 bis 37 C-Atomen,
SO der Rest C₆H₅-CH(-)-CH₂-O-
bedeutet.

2. Verfahren nach Anspruch 1, **dadurch gekennzeichnet, dass** als Si-H-Gruppen tragende Siloxane solche der Formel (I) 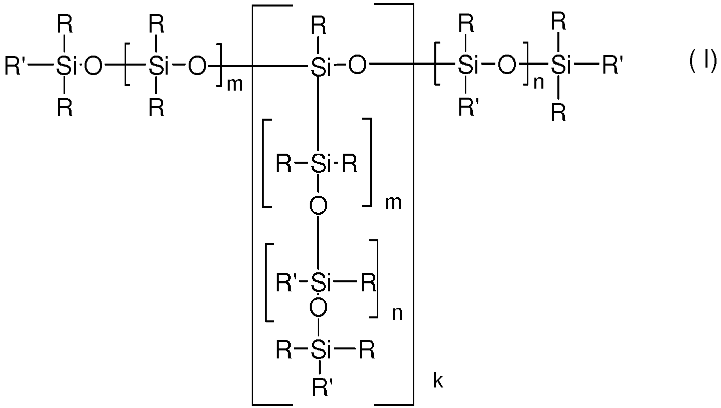 worin
R ein substituierter oder unsubstituierter Kohlenwasserstoffrest mit 1 bis zu 20 C-Atomen ist,
R' Wasserstoff und/oder R ist,
m 0 bis 500,
n 0 bis 60,
k 0 bis 10 ist,
mit der Maßgabe, dass R' mindestens einmal Wasserstoff ist eingesetzt werden.

3. Verfahren nach Anspruch 2, **dadurch gekennzeichnet, dass** der Rest R der Formel (I) ausgewählt ist aus einer oder mehreren gleiche oder verschiedene Gruppen der Alkylgruppen mit 1 bis 8 Kohlenstoffatomen, substituierte Alkylgruppen mit 1 bis 8 Kohlenstoffatomen, 3-Chlorpropyl-, 1 -Chlormethyl-, 3-Cyanopropylgruppe, Arylgruppen, Phenylgruppe, Aralkylgruppen mit 7 bis 20 Kohlenstoffatomen, Benzylgruppe, Alkoxy- oder Alkoxyalkylgruppen, Ethoxy- oder Ethoxypropylgruppe.

## Claims

1. Process for preparing reaction products from siloxanes having SiH groups and organic compounds bearing olefinic double bonds, **characterized in that** di-µ-chlorobis(1,2-η)cyclohexeneplatinum(II) chloride is used as a catalyst and **in that** a reaction is carried out without solvent and without auxiliary phases which compatibilize or dissolve the catalyst and the reaction takes place in neat form, wherein the olefinically unsaturated organic compounds are selected from the group of the α-olefins, the strained ring olefins, the α,ω-alkenols, the terminally olefinically unsaturated polyethers, the amino-functional α-olefins or the oxiranes bearing α-olefin groups, or the carboxylic esters olefinically unsaturated in the ω position, where the olefins can be used alone or in any desired mixtures with one another,
where terminally olefinically unsaturated polyethers are understood to mean those polyoxyalkylene compounds whose unsaturated terminus is defined by a vinyl, allyl or methallyl group,
selected from polyoxyalkylene compounds of the formulae
CH₂=CH-CH₂-O-(CH₂-CH₂O-)ₓ-CH₂-C (R')O-)_{y}(SO)_{z}-R'' (IV)
CH₂=CH-O-(CH₂-CH₂O-)ₓ-(CH₂-CH(R')O-)_{y}-R'' (V)
CH₂=CH-CH₂-R^{IV} (VI)
CH₂=CH-(O)_{x'}-R^{IV} (VII)
in which
x = 0 to 100,
x' = 0 or 1,
y = 0 to 100,
z = 0 to 100,
R' is an optionally substituted alkyl group having 1 to 4 carbon atoms and
R'' is a hydrogen radical or an alkyl group having 1 to 4 carbon atoms; the -C(O)-R''' group where R' ' ' = alkyl radical; the -CH₂-O-R' group; an alkylaryl group, such as the benzyl group; the -C(O)NH-R' group,
R^{IV} is an optionally substituted hydrocarbyl radical having 7 to 47 and preferably 13 to 37 carbon atoms,
SO is the C₆H₅-CH(-)-CH₂-O-radical.

2. Process according to Claim 1, **characterized in that** the siloxanes bearing Si-H groups used are those of the formula (I) 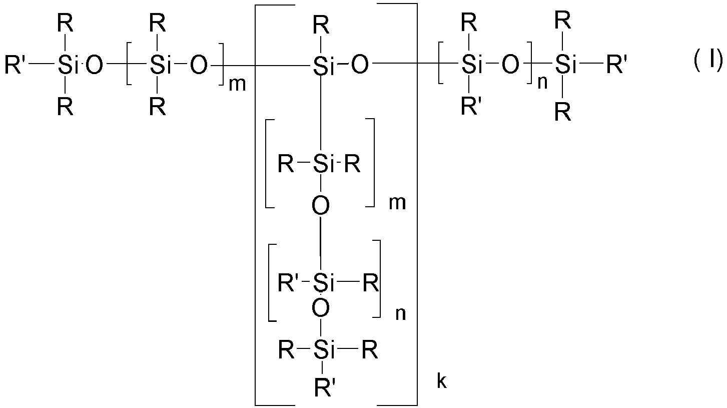 in which
R is a substituted or unsubstituted hydrocarbyl radical having 1 up to 20 carbon atoms,
R' is hydrogen and/or R,
m is 0 to 500,
n is 0 to 60,
k is 0 to 10,
with the proviso that at least one R' is hydrogen.

3. Process according to Claim 2, **characterized in that** the R radical of the formula (I) is selected from one or more identical or different groups from the alkyl groups having 1 to 8 carbon atoms, substituted alkyl groups having 1 to 8 carbon atoms, 3-chloropropyl, 1-chloromethyl, 3-cyanopropyl group, aryl groups, phenyl group, aralkyl groups having 7 to 20 carbon atoms, benzyl group, alkoxy or alkoxyalkyl groups, ethoxy or ethoxypropyl group.

## Revendications

1. Procédé pour la préparation de produits de transformation à partir de siloxanes présentant des groupes SiH et de composés organiques portant des doubles liaisons oléfiniques, **caractérisé en ce que** du di-µ-chloro-bis(1,2-η)-cyclohexène-chlorure de platine (II) est utilisé comme catalyseur et **en ce qu'**une réaction sans solvant et sans phases auxiliaires compatibilisant ou dissolvant le catalyseur est réalisée et la réaction a lieu dans la masse,
les composés organiques oléfiniquement insaturés étant choisis dans le groupe des α-oléfines, des oléfines cycliques tendues, des α,ω-alcénols, des polyéthers à terminaisons oléfiniquement insaturées, des α-oléfines à fonctionnalité amino ou des oxiranes portant des groupes α-oléfiniques ou des esters d'acides carboxyliques oléfiniquement insaturés en position ω, les oléfines pouvant être utilisées seules ou dans des mélanges quelconques les unes avec les autres
les polyéthers à terminaisons oléfiniquement insaturées étant des composés de polyoxyalkylène dont la terminaison insaturée est définie par un groupe vinyle, allyle ou méthallyle, choisis parmi les composés de polyoxyalkylène des formules
CH₂=CH-CH₂-O-(CH₂-CH₂O-)ₓ-(CH₂-CH(R')O-)_{y}(SO)_{z}-R" (IV)
CH₂=CH-O-(CH₂-CH₂O-)ₓ-(CH₂-CH(R')O-)_{y}-R" (V)
CH₂=CH-CH₂-R^{IV} (VI)
CH₂=CH-(O)_{x'}-R^{IV} (VII)
dans lesquelles
x = 0 à 100,
x' = 0 ou 1,
y = 0 à 100,
z = 0 à 100,
R' signifie un groupe alkyle le cas échéant substitué comprenant 1 à 4 atomes de carbone et
R" signifie un radical hydrogène ou un groupe alkyle comprenant 1 à 4 atomes de carbone ; le groupe -C(O)-R'" où R"' = radical alkyle ; le groupe -CH₂-O-R' ; un groupe alkylaryle, tel que le groupe benzyle ; le groupe -C(O)NH-R',
R^{IV} signifie un radical hydrocarboné le cas échéant substitué comprenant 7 à 47, de préférence 13 à 37 atomes de carbone,
SO signifie le radical C₆H₅-CH(-)-CH₂-O-.

2. Procédé selon la revendication 1, **caractérisé en ce qu'**on utilise, comme siloxanes portant des groupes SiH, ceux de formule (I) 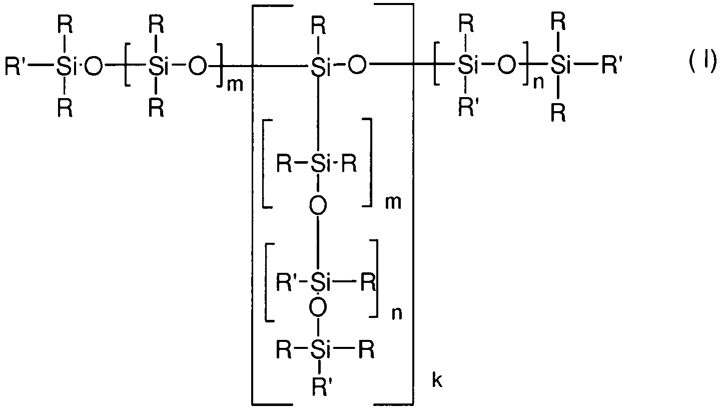 dans laquelle
R représente un radical hydrocarboné substitué ou non substitué comprenant 1 à 20 atomes de carbone,
R' représente hydrogène et/ou R,
m vaut 0 à 500,
n vaut 0 à 60,
k vaut 0 à 10,
à condition que R' soit au moins une fois de l'hydrogène.

3. Procédé selon la revendication 2, **caractérisé en ce que** le radical R de la formule (I) est choisi parmi un ou plusieurs groupes identiques ou différents parmi les groupes alkyle comprenant 1 à 8 atomes de carbone, les groupes alkyle substitué comprenant 1 à 8 atomes de carbone, le groupe 3-chloropropyle, le groupe 1-chlorométhyle, le groupe 3-cyanopropyle, les groupes aryle, le groupe phényle, les groupes aralkyle comprenant 7 à 20 atomes de carbone, le groupe benzyle, les groupes alcoxy ou alcoxyalkyle, le groupe éthoxy ou le groupe éthoxypropyle.
